# EUROPÄISCHE PATENTSCHRIFT

(11) **EP 0 616 031 B1**
(45) Veröffentlichungstag und Bekanntmachung des Hinweises auf die Patenterteilung: **19.06.2002**
(21) Anmeldenummer: 94102612.2
(22) Anmeldetag: 22.02.1994
(51) Int. Cl.: C12N 5/06, C12N 7/00, A61K 39/12

(54) **Rinderovarzellinie (FROv) zur Virusvermehrung**
Bovine ovary cell line (FROv) for virus multiplication
Lignée cellulaire de l'ovaire bovin (FROv) pour la multiplication de virus

(30) Priorität: 13.03.1993 DE 4308092
(43) Veröffentlichungstag der Anmeldung: 21.09.1994
(73) Patentinhaber: Aventis Behring Gesellschaft mit beschränkter Haftung, 35002 Marburg (DE)
(72) Erfinder: Bernhardt, Dieter, D-35091 Cölbe (DE)

(56) Entgegenhaltungen:
- WO-A-90/01337
- US-A- 4 070 453
- CANADIAN JOURNAL FOR COMPARATIVE MEDICINE, Bd. 49, Nr. 1, Januar 1985 Seiten 91-94, CHEN K.S.

## Beschreibung

Die Erfindung betrifft eine Rinderovarzellinie (FROv), ein Verfahren zu ihrer Herstellung und ihre Verwendung zur Vermehrung von Viren.

Zur Produktion von Proteinen und Virusantigenen werden lebende Zellen benötigt. Es ist Stand des Wissens hierfür diploide Zellstämme oder permanente Zellinien zu verwenden, sofern sie für die Produktion der gewünschten Proteine oder Virusantigene geeignet sind. Ebenso ist bekannt, daß im allgemeinen diploide Zellstämme für die meisten Virusarten besser empfindlich sind als permanente Zellinien, weshalb sie für die Vermehrung einer Vielzahl von Virusarten besonders geeignet sind.

Permanente Zellinien haben jedoch gegenüber diploiden Zellstämmen den Vorteil, daß sie ein unbegrenztes Wachstum besitzen, d.h. sie sind immortalisiert. In weiten Passagebereichen weisen solche permanenten Zellinien gleichbleibende Eigenschaften hinsichtlich der Zell- und Antigenvermehrung auf, da bei ihnen keine Zellenddifferenzierung wie bei diploiden Zellstämmen stattfindet. Neben der begrenzten Lebensspanne (Passagezahl) diploider Zellstämme, ist ein weiterer schwerwiegender Nachteil, daß die Organe, die als Ausgang für diploide Zellstämme benötigt werden, nicht in ausreichender Menge und zu jeder Zeit zur Verfügung stehen. Weiterhin kann das Ausgangsmaterial, die Organe, latent kontaminiert (Virus, Mycoplasmen, Bakterien) sein, wodurch eine optimal reproduzierbare Antigenproduktion nicht gewährleistet ist. Aus dieser vielschichtigen Problematik ist das Bestreben zu verstehen, für die Produktion von Viren, Virusantigenen sowie deren Nachweis (Titrationen), permanente Zellinien einzusetzen.

Auch vom Rind stammende permanente Zellinien sind bereits etabliert, beispielsweise MDBK, DBK, Aubeck (Rindernierenzellen) oder embryonale Schilddrüsenzellen. Der Nachteil dieser Zellinien ist aber, daß sich nur einige wenige Virusarten, wie IBR-, PI₃, BAV des Typs 1 oder 2 in ihnen vermehren lassen oder daß sie z. T. mit Fremdagenzien kontaminiert sind und somit für eine kommerzielle Antigenproduktion nicht optimal genutzt werden können.

Gegenstand der Erfindung ist eine permanente Zellinie aus fetalen Rinderovarien, worin sich überraschenderweise eine Vielzahl von Virusarten wie Bovine Adenoviren (BAV): Typ 1, 3, 4, 5, 6, 7, 8; Parainfluenzavirus (PI): Typ 3; Bovines-Respiratory syncytial Virus (BRSV); Bovine Herpesviren: Typ 1, 4; Equine Herpesviren: Typ 1; Mucosal disease Virus: Stamm Ug59; Bovines Parvovirus: Stamm Haden vermehren lassen. Diese Zellinie ist sowohl für die Produktion von Virusantigenen als auch für den Virusnachweis geeignet.

### Etablierung der fetalen - Rinder-Ovar-Zellinie (FROv)

### Ausgang:

Einem etwa 7 Monate alten Fetus von einem schwarzbunten weiblichen Rind vom Schlachthof Marburg vom 14.11.1990 wurden unter sterilen Bedingungen die Ovarien entnommen. Die Ovarien wurden zerkleinert und bei RT mit Trypsin in Einzelzellen aufgeschlossen. Nach Abschluß der Trypsinbehandlung wurde die Zellsuspension zentrifugiert, das Zellsediment in Eagle Minimal Essential Medium (EMEM) resuspendiert (gewaschen) und erneut zentrifugiert.

### Zellanzucht:

Das zentrifugierte, gewaschene Zellsediment wurde in EMEM + 5 % FKS + Antibiotika

| | |
|---|---|
| Neomycin (reine Base) | 0.2625 g/l |
| Streptomycin (reine Base) | 0.2565 g/l und |
| Penicillin | 0.1212 g/l; |

(NSP) im Verhältnis 1:300 aufgenommen und in T50 Flaschen (Fa. Greiner, Art. Nr. 690 160) ausgesät.

### Zellzucht bis zur 35. Passage

Die FROv-Zellen wurden bis zur 35. Passage in EMEM + 5 % FKS + NSP gezüchtet. Nach Dichtwachsen wurden die Kulturen 1:5 umgesetzt (ca. 4-6 Tage). Während der Zellpassagen veränderte sich die Kultur vom gemischten Zelltyp (Fibroblasten, polygonale Zellen) zu einem mehr einheitlichen polygonalen Zelltyp.

### Zellzucht nach der 35. Passage

Die FROv-Zellen wurden auf drei verschiedene Weisen weiter passagiert (Zweige A, B und C):
Zweig A der FROv-Zellen wurde mit EMEM + 5 % FKS + (NSP) bis zur 56. Passage gezüchtet. Die Splittingrate betrug 1:5 bis 1:7.

Die 55. Passage wurde unter der Hinterlegungsnummer DSM ACC2051 bei der DSM - Deutsche Sammlung von Mikroorganismen und Zellkulturen GmbH, Mascheroder Weg 1 B, 3300 Braunschweig hinterlegt.

Von der 56. Passage an wurde der Speziesidentitätstest mittels Isoenzymmuster durchgeführt. Ergebnis: Spezies Rind.

Zweig B wurde ab der 35. Passage mit EMEM + 2.5 % FKS + 2.5 % Pferdeserum + NSP vermehrt. In der 45. Passage und 51. Passage wurden FROv-Zellen in flüssigem N₂-eingefroren. Derzeit befinden sich die FROv-Zellen vom Zweig B in der 105. Passage. Die Splittingrate betrug 1:5 bis 1:10.

Zweig C der FROv-Zellen wurde aus der 51. Passage von Zweig B nach Lagerung der Zellen in flüssigem N₂-etabliert. Nach Auftauen der Zellsuspension wurden die Zellen viermal passagiert. Von der 55. Passage wurde ein master cell stock (MCS) angelegt.

Hierfür wurde die Zellsuspension auf 1.8 x 10⁶ Zellen/ml eingestellt und in Teflonampullen zu 1.0 ml abgefüllt und in flüssigem N₂-gelagert. Der MCS wurde auf Sterilität und auf Freisein von Mycoplasmen und Fremdvirus geprüft.

### Ergebnis: Der MCS ist steril, frei von Mycoplasmen und Fremdvirus

8 Tage nach dem Einfrieren des MCS wurde eine Ampulle dem flüssigen N₂-entnommen, aufgetaut, und in einer 250 ml Kulturschale der Fa. Greiner, Art Nr. 6458170, in Züchtungsmedium ausgesät und zur weiteren Passagierung bei 37°C angezüchtet. Die Züchtung der FROv-Zellen im Zweig C erfolgte in EMEM + 2.5 % FKS + 2.5 % Pferdeserum + NSP.
Die hier angegebene Züchtung der FROv Zellen ist beispielhaft. Die Kultivierung der Zellen in anderen erprobten Medien (wie DMEM, TCM199) und mit unterschiedlichen Seren (Kälberserum, newborn calf Serum, Schafserum) zwischen 1 und 10 % ist ebenso möglich.

Prüfung der Virusvermehrung in FROv-Zellen (Zweig B)
Ab der 69. Passage wurde die Empfänglichkeit der FROv-Zellinie, Zweig B, gegenüber folgenden Virusarten geprüft:
Bovine Adenoviren: Typ 1, 3, 4, 5, 6, 7, 8
Parainfluenzavirus: Typ 3
Bovines-Respiratory syncytial Virus (BRSV)
Bovine Herpesviren: Typ 1, 4
Equine Herpesviren: Typ 1
Mucosal disease Virus (MDV): Stamm Ug59
Bovines Parvovirus: Stamm Haden

### Beispiel 1

Dichtgewachsene FROv-Zellkulturen (T50 Flaschen, Fa. Greiner) wurden auf EMEM + NSP gewechselt, um das serumhaltige Anzuchtmedium zu entfernen. Danach wurden die Kulturen mit den genannten Virusarten in serumfreien Medien infiziert.
Bei bovinem Parvovirus erfolgte die Infektion bereits nach Auswachsen der Kultur von 70-80 %. Drei nicht infizierte Kulturen wurden bei diesem Infektionsversuch als Kontrollen mitgeführt.

Nach 3-10 Tagen zeigten die infizierten Kulturen, in Abhängigkeit von der Virusart, einen virusspezifischen, zytopathogenen Effekt. In den FROv-Zellen hatte eine Virusvermehrung stattgefunden. Hingegen wiesen die nichtinfizierten Kontrollkulturen einen geschlossenen, unveränderten Zellrasen auf.

### Beispiel 2

Um die Virussynthese der FROv-Zellinie in den verschiedenen Passagen zu überprüfen, wurden die nachfolgend tabellarisch aufgeführten Passagen der FROv-Zellen mit BVD-Stamm Ug59 infiziert. Bei der Infektion wurde in gleicher Weise wie in Beispiel 1 verfahren. Die Ergebnisse beinhaltet die folgende Tabelle.

| **BVD-Stamm Ug59 Vermehrung in der FROv-Zellinie in verschiedenen Passagen** | | |
|---|---|---|
| Passage der FROv-Zellen | Virusernte, in Tagen p.i | Virustiter in FRLu 45 Zellen/ml der Virusernte |
| 69 | 4 | 10^{5.7} |
| 70 | 5 | 10^{5.3} |
| 72 | 5 | 10^{6.1} |
| 75 | 4 | 10^{6.3} |
| 75 | 4 | 10^{6.1} |
| 75 | 4 | 10^{6.1} |
| 75 | 4 | 10^{6.5} |
| 78 | 5 | 10^{6.1} |
| 80 | 4 | 10^{5.9} |
| 81 | 4 | 10^{6.1} |
| 83 | 5 | 10^{6.1} |
| 88 | 5 | 10^{5.8} |
| 95 | 5 | 10^{6.7} |
| 100 | 4 | 10^{6.1} |
| x*) | | 10^{6.1} |

| | | |
|---|---|---|
| *) geometrischer Mittelwert | | |

Zum Vergleich wurde die Virusvermehrung von BVD-Stamm Ug59 in diploiden FRLu45-Zellen überprüft und hierbei die nachfolgend aufgeführten Werte festgestellt:

| Passage der FRLu-Zellen | Virusernte in Tagen p.i | Virustiter in log10 in FRLu 45 Zellen/ml der Virusernte |
|---|---|---|
| 5 | 4 | 5.6 |
| 6 | 3 | 6.5 |
| 8 | 5 | 6.7 |
| 10 | 5 | 5.6 |
| 10 | 4 | 5.3 |
| 11 | 3 | 5.8 |
| 11 | 5 | 5.6 |
| x | | 5.9 |

Ergebniszusammenfassung: Wie aus den Ergebnissen in den beiden Tabellen hervorgeht, ist die produzierte BVD-Antigenmenge (Infektiosität) in beiden Zellsystemen vergleichbar.

### Beispiel 3

BVD-Stamm Ug59-Virusernten wurden um die Virussensitivität der FROv-Zellen zu prüfen, vergleichend in FRLu 45- und FROv-Zellen titriert. Die Bestimmung der Infektiositätstiter erfolgte als Doppeltitration.

| Vergleichstitration von BVD-Ug59 in FROv- und FRLu 45-Zellen | | |
|---|---|---|
| Virusernte | Titer in log10/ml in FROv-Zellen | Titer in log10/ml in FRLu 45-Zellen |
| 1 | 5.3 | 5.6 |
| 2 | 6.3 | 6.5 |
| 3 | 5.8 | 5.7 |
| 4 | 5.6 | 5.8 |
| 5 | 5.0 | 5.6 |
| 6 | 5.3 | 5.3 |
| 7 | 6.0 | 6.7 |
| 8 | 5.4 | 5.4 |
| 9 | 5.1 | 5.3 |
| 10 | 5.1 | 5.1 |
| 11 | 5.6 | 5.8 |
| 12 | 5.3 | 5.6 |
| 13 | 5.5 | 5.3 |
| 14 | 5.1 | 5.6 |
| 15 | 5.2 | 5.8 |
| 16 | 1.3 | 1.7 |
| x | 5.2 | 5.4 |

Zusammenfassung: Wie diese Vergleichsuntersuchungen zeigen, ist die FRLu 45-Zelle nur unwesentlich empfindlicher beim BVD-Virusnachweis als die FROv-Zellinie.

## Patentansprüche

1. Permanente Zellinie aus fetalen Rinderovarien mit den Eigenschaften einschließlich der Virusempfindlichkeit der unter der Nummer DSM ACC2051 bei der Deutschen Sammlung für Mikroorganismen (DSM) hinterlegten Zellinie.

2. Zellinie nach Anspruch 1 hinterlegt bei der DSM unter der Nummer DSM ACC2051.

3. Zellinie nach Anspruch 1 mit den Wachstumscharakteristiken der Zellinie gemäß Anspruch 2.

4. Zellinie nach Anspruch 1, **dadurch gekennzeichnet, daß** sich Bovine Adenoviren (BAV) Typ 1, 3, 4, 5, 6, 7, 8 Parainfluenzavirus (PI) Typ 3, Bovines-Respiratory syncytial Virus (BRSV), Bovine Herpesviren Typ 1, 4, Equine Herpesviren Typ 1, Mucosal disease Virus Stamm Ug59 oder Bovines Parvovirus Stamm Haden auf ihr vermehren lassen.

5. Verwendung einer Zellinie gemäß Anspruch 1 zur Produktion von Virusantigenen.

## Claims

1. A permanent cell line comprising fetal bovine ovaries having the properties, including the sensitivity to viruses, of the cell line deposited with the Deutsche Sammlung für Mikroorganismen (DSM) (German collection for microorganisms) under the number DSM ACC2051.

2. The cell line as claimed in claim 1, deposited with the DSM under the number DSM ACC2051.

3. The cell line as claimed in claim 1, having the growth characteristics of the cell line as claimed in claim 2.

4. The cell line as claimed in claim 1, which supports the replication of bovine adenoviruses (BAV) types 1, 3, 4, 5, 6, 7 and 8, parainfluenzavirus (PI) type 3, bovine respiratory syncytial virus (BRSV), bovine herpesviruses types 1 and 4, equine herpesviruses type 1, mucosal disease virus strain Ug59 or bovine parvovirus strain Haden.

5. The use of a cell line as claimed in claim 1 for producing viral antigens.

## Revendications

1. Lignée cellulaire permanente provenant d'ovaires bovins foetaux, ayant les propriétés, y compris la sensibilité virale, de la lignée cellulaire déposée sous le numéro DSM ACC2051 à la Deutsche Sammlung für Mikroorganismen (DSM).

2. Lignée cellulaire selon la revendication 1, déposée à la DSM sous le numéro DSM ACC2051.

3. Lignée cellulaire selon la revendication 1, ayant les caractéristiques de croissance de la lignée cellulaire selon la revendication 2.

4. Lignée cellulaire selon la revendication 1, **caractérisée en ce que** les adénovirus bovins (BAV) types 1, 3, 4, 5, 6, 7, 8, le virus para-influenza (PI) type 3, le virus syncytial respiratoire bovin (BRSV), les herpesvirus bovins types 1, 4, l'herpesvirus équin type 1, le virus de la maladie des muqueuses souche Ug59 ou le parvovirus bovin souche Haden peuvent se multiplier sur elle.

5. Utilisation d'une lignée cellulaire selon la revendication 1, pour la production d'antigènes viraux.
